Europäisches Patentamt

(19) European Patent Office

Office européen des brevets

(11) Publication number: **0 013 187**
**B1**

(12) EUROPEAN PATENT SPECIFICATION

(45) Date of publication of patent specification: **15.06.83**

(21) Application number: **79303082.6**

(22) Date of filing: **31.12.79**

(51) Int. Cl.³: **A 61 F 9/00**

(54) Eyedropper-bottle holder.

(30) Priority: **02.01.79 US 37**
**31.07.79 US 62585**

(43) Date of publication of application:
**09.07.80 Bulletin 80/14**

(45) Publication of the grant of the patent:
**15.06.83 Bulletin 83/24**

(84) Designated Contracting States:
**AT BE CH DE FR GB IT LU NL SE**

(56) References cited:
**DE - B - 2 362 147**
**DE - B - 2 447 872**
**US - A - 2 676 592**
**US - A - 2 722 216**
**US - A - 3 058 466**
**US - A - 3 439 674**
**US - A - 3 521 636**
**US - A - 3 934 590**

(73) Proprietor: **MERCK & CO. INC.**
**126, East Lincoln Avenue P.O. Box 2000**
**Rahway New Jersey 07065 (US)**

(72) Inventor: **Gibilisco, Kenneth J.**
**674 Joseph Road**
**Warminster Pennsylvania 18974 (US)**

(74) Representative: **Purvis, William Michael Cameron et al,**
**D. Young & Co. 10 Staple Inn**
**London WC1V 7RD (GB)**

Courier Press, Leamington Spa, England

## Eyedropper-bottle holder

The invention relates to an eyedropper-bottle holder and stabilizer for administering eyedrops from an ordinary squeeze-type dropper bottle.

An object of the invention is the provision of a bottle holder that can be used interchangeably with bottles of the same or different sizes, and which can be adjusted to suit an individual patient, allowing safe and accurate administration of drops to the eye of the patient.

In the administration of eyedrops, it is important to hold the dispensing tip steady and far enough from the eye to allow the medication to drop directly onto the eyeball and to keep the tip of the dropper-bottle from touching the eye. It is important and desirable to drop the medication near the centre of the eye, and to be able to do this using only one hand. In self-administration of eyedrops, it is necessary to brace that hand against a steady object and at the same time position the dropper-bottle tip directly over the centre of the eye. It is desirable, if possible, to position the tip of the dropper-bottle directly over the centre of the eye so that the eye receiving the medication can see the drop as it forms and falls.

The ordinary eyedroppers or squeeze-type dropping bottles are convenient: simple to operate, and inexpensive to manufacture. However, such devices suffer from the drawback of messiness and unsteadiness of the person who is self-administering the medication. A number of devices have been proposed to allow steady, safe and accurate dispensing.

USA specification 2 676 592 shows a device with a nose-bridge rest at one end of a stepped member, the other end of which member is formed as a handle and an intermediate portion of which has an opening therein or is formed as a frusto-conical tube to receive an eyedropper and position it above the eye into which drops are to be inserted.

USA specification 3 521 636 shows a nose-bridge rest formed as an integral part of the administering container; and thus when the medication is exhausted, the relatively expensive device must be discarded along with the container.

USA specification 3 439 674 shows an eyewash dispenser having an eyelid engaging means adjustably mounted thereon for use for pressing an eyelid to an open position while the eyewash is being administered.

According to the invention there is provided an eyedropper-bottle holder for dispensing eyedrops comprising means to receive an eyedropper-bottle of the squeeze-type and a nose-bridge rest incorporating a spacer bar provided at one end with a depressed centre portion for fitting on the nose-bridge of the user, wherein the spacer bar at its other end is provided with a slide channel extending transversely to the longitudinal direction of the spacer bar, in which slide channel a slide bar can be received, the cooperating opposed surfaces of the slide bar and of the slide channel being provided with cooperating transverse ridges to define a plurality of optionally usable stable positions of the slide bar relative to the slide channel and wherein the means to receive an eyedropper-bottle comprises an opening in one end of the slide bar circumscribed by a notched resilient collar, whereby the spacing of the opening with an eyedropper-bottle received therein from the nose-bridge rest is adjustable to suit the spacing of the centre of the eye from the nose of a user.

The holder can be used with a standard size and type dropper-bottle, and can be used merely by inserting the inexpensive dropper-bottle into the holder and using it in the manner subsequently described herein.

A further advantage of the invention is that it may be used with various sizes and types of eyedropper-bottle, and can be used by merely inserting the inexpensive dropper-bottle into the holder, adjusting the slide bar in the slide channel, and using it in the manner subsequently described herein. In addition to being an inexpensive and simple device, the present holder can be lighter than the devices of the prior art and therefore much easier to control by a patient administering medication to himself.

The invention is diagrammatically illustrated by way of example in the accompanying drawings, in which:—

Figure 1 is a perspective view showing use of an eyedropper-bottle holder according to the invention by a patient;

Figure 2 is a sectional view taken on line 2—2 of Figure 1 showing the eyedropper-bottle holder with an eyedropper-bottle positioned ready for use;

Figure 3 is a horizontal sectional view taken on line 3—3 of Figure 2; and

Figure 4 is a perspective view of the two component parts of the eyedropper-bottle holder of Figures 1 to 3 before assembly.

A first component of the holder comprises a flat spacer bar 31 terminating in the nose-bridge rest 30, which is of concave construction with a depressed centre portion 29 so that it can rest in a stable position on the bridge of the nose of a patient. At the other end of the spacer bar 31 there is a slide 32 defining a rectangularly shaped slide channel 33.

A second component of the holder comprises a flat slide bar 34 of rectangular cross-sectional and having at one end a circular-shaped extension 35 integral with the flat slide bar 34, the extension 35 having an opening therein to receive the neck of an eyedropper-bottle 24. The opening is surrounded by an integral collar 36 having slits 37 cut through the collar at regular intervals. The collar 36 is designed to provide a snug fit for the neck of dropper bottles of various sizes, thus providing a versatile bottle holder.

The slide bar 34 is inserted in the slide channel 33 and before use the relative position of the slide bar 34 in the slide channel 33 is adjusted until the horizontal displacement between the depressed centre 29 of the nose-bridge 30 and a tip 26 of the dropper bottle 24 matches the horizontal distance between the patient's nose bridge and the pupil of the eye being treated.

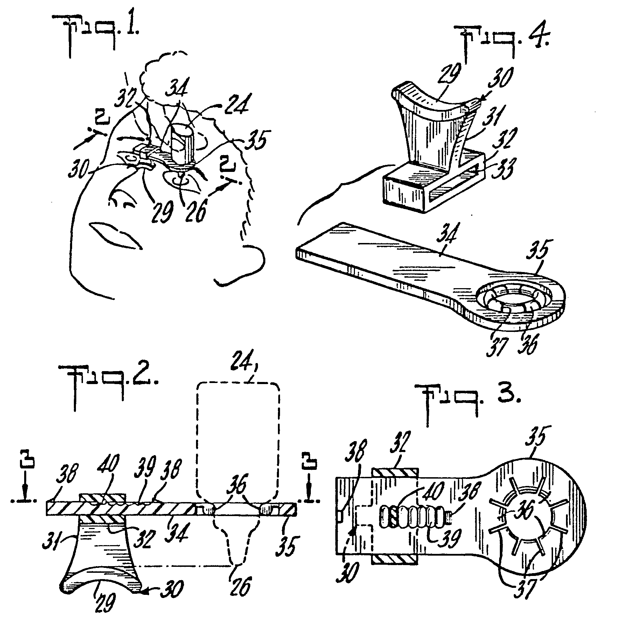

In use, as shown in Figure 1, the head of the patient is tilted back and the holder and a dropper bottle held therein is inverted and positioned so that the dropper-bottle tip is positioned directly over the pupil of the eye being treated.

So that the slide bar 34 can be held in any one of several positions, the surface of the slide bar 34 which is uppermost in the position of use has at least one stop 38 to prevent the slide 32 from slipping off the end of the slide bar 34 or coming into contact with the dropper bottle 24. Said surface is further provided with spaced, transverse ridges 39 to engage at any one of various stop positions with a raised transverse ridge 40 on the co-operating opposing internal surface of the slide channel 33. The vertical spacer bar 31 carrying the nose-bridge rest 30 is designed so that the depressed centre 29 of the nose-bridge 30 is slightly less further displaced vertically from the top surface of the slide bar 34 than is the tip 26 of the dropper bottle 24.

The entire holder is fabricated of a resilient translucent or opaque plastics material such as vinyl or low-density polyethylene, polypropylene or nylon.

## Claim

An eyedropper-bottle holder for dispensing eyedrops comprising means to receive an eyedropper-bottle (24) of the squeeze-type and a nose-bridge rest (30) incorporating a spacer bar (31) provided at one end with a depressed centre portion (29) for fitting on the nose-bridge of the user characterised in that the spacer bar (31) at its other end is provided with a slide channel (33) extending transversely to the longitudinal direction of the spacer bar (31), in which slide channel (33) a slide bar (34) can be received, the cooperating opposed surfaces of the slide bar and of the slide channel (33) being provided with cooperating transverse ridges (39, 40) to define a plurality of optionally usable stable positions of the slide bar (34) relative to the slide channel (33) and in that the means to receive an eyedropper-bottle comprises an opening in one end of the slide bar (34) circumscribed by a notched resilient collar (36), whereby the spacing of the opening (36) with an eyedropper-bottle (24) received therein from the nose-bridge rest is adjustable to suit the spacing of the centre of the eye from the nose of a user.

## Revendication

Support pour bouteille compte-gouttes ophtalmiques pour distribuer des gouttes ophtalmiques comprenant un dispositif pour recevoir une bouteille compte-gouttes ophtalmiques (24) du type compressible et un appui (30) se plaçant sur l'arête du nez comprenant une barre d'espacement (31) munie à une extrémité d'une portion centrale déprimée (29) pour s'adapter à l'arête du nez de l'utilisateur, caractérisé en ce que la barre d'espacement (31) à son autre extrémité est munie d'un canal de glissement (33) s'étendant transversalement à la direction longitudinale de la barre d'espacement (31), ce canal de glissement (33) pouvant recevoir une barre coulissante (34), les surfaces opposées coopérantes de la barre coulissante et du canal de glissement (33) étant munies d'arêtes transversales coopérantes (39, 40) pour déterminer plusieurs positions stables utilisables au choix de la barre coulissante (34) par rapport au canal de glissement (33) et en ce que le dispositif pour recevoir une bouteille compte-gouttes ophtalmiques est constitué d'une ouverture dans une extrémité de la barre coulissante (34) délimitée par un collier (36) élastique encoché, si bien que la distance entre l'ouverture (36) munie d'une bouteille compte-gouttes ophtalmiques (24) et l'appui se plaçant sur l'arête, du nez est réglable pour s'adapter à la distance séparant le centre de l'oeil du nez d'un utilisateur.

## Patentanspruch

Ein Augentropferflaschenhalter für die Verabreichung con Augentropfen, umfassend Einrichtungen für die Aufnahme einer Augentropferflasche (24) vom Quetschtypus und eine Nasenrückenauflage (30), welche einen an einem Ende mit einem vertieften Mittelteil (29) für den Sitz auf dem Nasenrücken des Benützers versehenen Abstandhaltersteg (31) aufweist, dadurch gekennzeichnet, daß der Abstandhaltersteg (31) an seinem anderen Ende mit einem Gleitkanal (33) versehen ist, der sich quer zur Längsrichtung des Abstandhaltersteges (31) erstreckt, in welchen Gleitkanal (33) eine Gleitschiene (34) aufgenommen werden kann, wobei die Zusammenwirkenden, entgegengesetzten Oberflächen der Gleitschiene und des Gleitkanals (33) mit zusammenwirkenden Querrippen (39, 40) versehen sind, die eine Mehrzahl von gewünschtenfalls brauchbaren, stabilen Positionen der Gleitschiene (34) relativ zum Gleitkanal (33) festlegen, und daß die Einrichtungen für die Aufnahme einer Augentropferflasche eine von einem gekerbten elastischen Kragen (36) eingefaßte Öffnung in einem Ende der Gleitschiene (34) umfassen, wodurch der Abstand der Öffnung (36) mit einer darin aufgenommenen Augentropferflasche (24) von der Nasenrückenauflage so einstellbar ist, daß er dem Abstand der Augenmitte von der Nase eines Benützers entspricht.

Fig. 1.

Fig. 4.

Fig. 2.

Fig. 3.